(19) **Europäisches Patentamt**
**European Patent Office**
**Office européen des brevets**

(11) **EP 3 424 449 A1**

(12) **EUROPEAN PATENT APPLICATION**
published in accordance with Art. 153(4) EPC

(43) Date of publication:
**09.01.2019 Bulletin 2019/02**

(21) Application number: **17759998.2**

(22) Date of filing: **28.02.2017**

(51) Int Cl.:
*A61B 17/58* [(2006.01)]     *A61L 27/04* [(2006.01)]
*A61L 27/40* [(2006.01)]

(86) International application number:
**PCT/JP2017/007787**

(87) International publication number:
**WO 2017/150532 (08.09.2017 Gazette 2017/36)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **29.02.2016 JP 2016038133**

(71) Applicant: **Medtronic Sofamor Danek, Co., Ltd.**
**Osaka-shi, Osaka 553-0003 (JP)**

(72) Inventors:
• **SUGINO, Atsushi**
  **Osaka-shi**
  **Osaka 553-0003 (JP)**
• **FUKUZAKI, Satoshi**
  **Tsu-shi**
  **Mie 514-8507 (JP)**

(74) Representative: **FRKelly**
**27 Clyde Road**
**Dublin D04 F838 (IE)**

(54) **ANTIMICROBIAL IN VIVO IMPLANTATION DEVICE**

(57) An antimicrobial implant device made of a first material and a second material, wherein at least a surface of the first material comprises an antimicrobial metal; at least a surface of the second material is nobler than the first material; an electric circuit is formed between the first material and the second material when the device is implanted in vivo for use; and ions of the antimicrobial metal are eluted from the first material under a presence of an electrolyte to impart an antimicrobial property to a surface and/or a surrounding area of the first material.

## Fig. 8

EP 3 424 449 A1

**Description**

TECHNICAL FIELD

The present invention relates to an implant device, and specifically relates to an implant device that achieves an antimicrobial property while it is implanted in vivo for use.

BACKGROUND ART

A variety of instruments such as cages, wires, plates, screws, rods, connectors, crosslinks, hooks, set screws, artificial vertebral bodies, and artificial intervertebral discs are used as orthopedic implant devices. These instruments may be contaminated with microorganisms when they are implanted in vivo for use. If an implant device is colonized by microorganisms, it is very difficult to prevent microbial growth. To prevent microbial growth and infection in the body, it is necessary to perform surgery to remove the implant device for replacement with a new one, which places an enormous burden on the patient. It is desired that an antimicrobial implant device be provided.

As a technique for imparting an antimicrobial property to an implant device, a medical implant system has been proposed (Patent Literature 1) which comprises a metal component containing an antimicrobial metal disposed on an external surface of the implant body; a power source having a first terminal and a second terminal, one of the terminals being in electrical communication with the metal component; and an insulator placed in a current path between the first terminal of the power source and the second terminal of the power source, preventing current flowing from the first terminal from reaching the second terminal without completing a circuit including a conductive body tissue or fluid adjacent to the external surface of the implant system when implanted. Patent Literature 1 discloses that examples of the antimicrobial metal include silver, copper, both silver and copper, both silver and cadmium, and a combination of silver, copper, and cadmium. In Patent Literature 1, the external power source equipment and the terminals are required in addition to the implant body to achieve an antimicrobial property, and a treatment or an external operation is needed to impart an antimicrobial property. Further improvement is required from the viewpoint of reducing the burden on patients.

CITATION LIST

PATENT LITERATURE

PTL 1: Japanese Patent No. 5175185

SUMMARY OF INVENTION

TECHNICAL PROBLEM

It is an object of the present invention to provide an implant device that can achieve an antimicrobial property, simply by being implanted in vivo, to reduce the burden on patients.

SOLUTION TO PROBLEM

Specific embodiments of the present invention are as set forth below:

[1] An antimicrobial implant device made of a first material and a second material, wherein
at least a surface of the first material comprises an antimicrobial metal;
at least a surface of the second material is nobler than the first material;
an electric circuit is formed between the first material and the second material when the device is implanted in vivo for use; and
ions of the antimicrobial metal are eluted from the first material under a presence of an electrolyte to impart an antimicrobial property to a surface and/or a surrounding area of the first material.
[2] The antimicrobial implant device according to [1], wherein the first material comprises at least one antimicrobial metal selected from cobalt, silver, zinc, copper, tungsten, magnesium, nickel and phosphorus.
[3] The antimicrobial implant device according to [1] or [2], wherein the first material is a cobalt-based alloy.
[4] The antimicrobial implant device according to [3], wherein the cobalt-based alloy comprises cobalt in an amount of 29 to 69 mass%.
[5] The antimicrobial implant device according to [3] or [4], wherein the cobalt-based alloy is a cobalt-chromium alloy.
[6] The antimicrobial implant device according to [5], wherein the cobalt-chromium alloy comprises chromium in an amount of 18 to 30 mass%.

[7] The antimicrobial implant device according to any one of [1] to [6], wherein the second material is titanium or a titanium-based alloy.

[8] The antimicrobial implant device according to [7], wherein the titanium or the titanium-based alloy comprises titanium in an amount of 68 to 100 mass%.

[9] The antimicrobial implant device according to any one of [1] to [8], wherein a ratio of a surface area of the second material to a surface area of the first material (second material/first material) is in a range of 0.2 to 10.8.

[10] The antimicrobial implant device according to any one of [2] to [9], wherein cobalt ions are eluted from the first material in an amount of 7 nmol/l to 81 $\mu$mol/l.

[11] The antimicrobial implant device according to any one of [1] to [10], wherein

the antimicrobial implant device is a screw comprising a screw portion and a head portion;

the head portion of the screw comprises the first material; and

the screw portion of the screw comprises the second material. The screw may be either of a fixed type in which the screw portion and the head portion are integrally formed, or a movable type in which the screw portion is movably joined to the head portion.

[12] The antimicrobial implant device according to [11], wherein the screw is a hollow screw having a hollow on the inside of the screw portion.

[13] The antimicrobial implant device according to [11] or [12], wherein the screw is a hollow screw having a hollow on the inside of the screw portion and a window that allows the hollow inside to communicate with the outside of the screw.

[14] The antimicrobial implant device according to any one of [11] to [13], wherein the screw portion comprises

a porous portion comprising the second material; and

a solid portion comprising the second material.

[15] The antimicrobial implant device according to any one of [1] to [10], wherein

the antimicrobial implant device is a screw comprising a screw portion and a head portion; and

the head portion of the screw comprises the second material; and

the antimicrobial implant device further has a set screw comprising the first material that is screwed on the head portion and/or a cap comprising the first material that is fit on the head portion.

[16] The antimicrobial implant device according to [15], wherein the head portion comprises

a porous portion comprising the second material; and

a solid section comprising the second material.

ADVANTAGEOUS EFFECTS OF INVENTION

The implant device of the present invention, once implanted in vivo, can achieve an antimicrobial property without requiring an external operation or treatment, thereby significantly reducing the burden on patients.

BRIEF DESCRIPTION OF DRAWINGS

Fig. 1 is an explanatory schematic diagram showing a state in which an antimicrobial implant device is used.

Fig. 2 is a perspective view of an antimicrobial implant device of an embodiment in the form of a screw.

Fig. 3 is a cross-sectional view of an antimicrobial implant device of an embodiment having a hollow-type screw portion.

Fig. 4 is a perspective view of an antimicrobial implant device of an embodiment having a hollow-type screw portion, and having windows that allow the hollow to communicate with the outside.

Fig. 5 is a cross-sectional view of the antimicrobial implant device of the embodiment having windows that allow the hollow to communicate with the outside shown in Fig. 4.

Fig. 6 is a cross-sectional view (lower left) of an antimicrobial implant device of an embodiment in which a screw portion has a solidified portion and sintered portions, along with a partially enlarged view (upper right) thereof.

Fig. 7 is a perspective view of an antimicrobial implant device of an embodiment in which a screw portion and a head portion are formed of the same alloy material, and a set screw screwed on the head portion is formed of a different alloy material.

Fig. 8 is a perspective view of an antimicrobial implant device of an embodiment in which a head portion is covered with a cap.

DESCRIPTION OF EMBODIMENTS

The present invention will be described with reference to the attached drawings.

An antimicrobial implant device of the present invention is an implant device made of a first material and a second material, wherein at least a surface of the first material comprises an antimicrobial metal; at least a surface of the second material is nobler than the first material; an electric circuit is formed between the first material and the second material when the device is implanted in vivo for use; and ions of the antimicrobial metal are eluted from the first material under a presence of an electrolyte to impart an antimicrobial property to a surface and/or a surrounding area of the first material. The antimicrobial implant device of the present invention is made of at least two materials, and simply because these two materials are contacted in vivo, a short circuit occurs to form an electric circuit.

Preferably, the first material comprises at least one antimicrobial metal selected from cobalt, silver, zinc, copper, tungsten, magnesium, nickel and phosphorus, and is preferably a cobalt-based alloy. More preferably, the cobalt-based alloy comprises cobalt in an amount of 29 to 69 mass%. Still more preferably, the cobalt-based alloy is a cobalt-chromium alloy comprising chromium in an amount of 18 to 30 mass%.

Preferably, the second material is titanium or a titanium-based alloy. More preferably, the titanium or the titanium-based alloy comprises titanium in an amount of 68 to 100 mass%.

More preferably, a ratio of a surface area of the second material to a surface area of the first material (second material/first material) = 0.2 to 10.8.

When the first material made of the cobalt-based alloy comprising cobalt in an amount of 29 to 69 mass% and the second material made of the titanium-based alloy comprising titanium in an amount of 68 to 100 mass% are used in combination, cobalt ions are eluted from the first material in an amount of 7 nmol/l to 81 $\mu$mol/l to provide a good antimicrobial property.

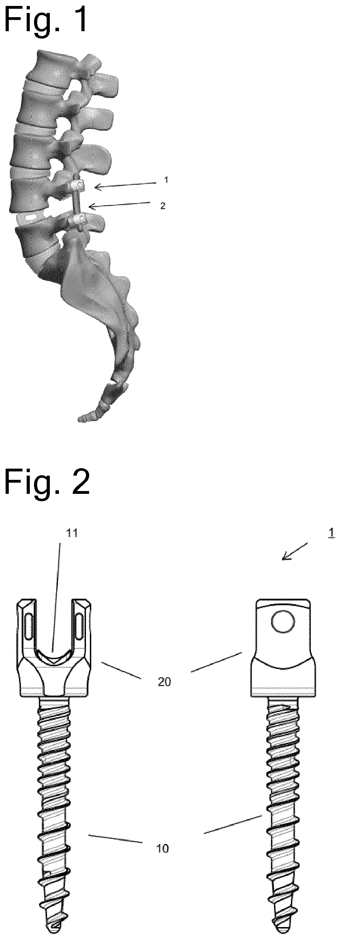

Fig. 1 is an explanatory schematic diagram showing a state in which an antimicrobial implant device of the present invention is used. Fig. 1 shows two screws 1, each of which is implanted in a vertebra (pedicle), and a rod 2 supported between these screws.

Fig. 2 is a perspective view of the screw 1 shown in Fig. 1, for example. The screw 1 has a screw portion 10 and a head portion 20. An upper end 11 of the screw portion 10, together with the head portion 20, supports the rod 2. At least a surface of the head portion 20 is made of a first material, and at least a surface of the screw portion 10 is made of a second material. At least a surface of the first material comprises an antimicrobial metal, at least a surface of the second material is nobler than the first material, and the first material and the second material are a combination of materials having a potential difference, such that the contact between the head portion 20 and the screw portion 10 causes a short circuit to form an electric circuit, and antimicrobial metal ions are eluted from the first material of the head portion 20 under the presence of an electrolyte to impart an antimicrobial property to the surface and/or a surrounding area of the head portion 20.

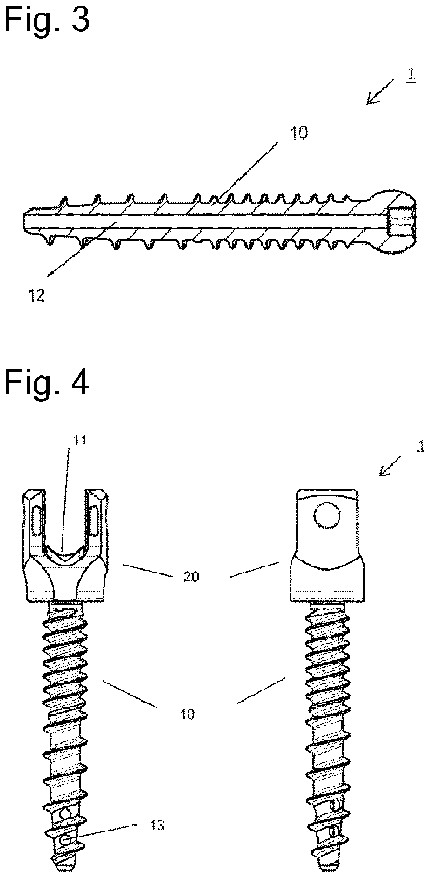

Fig. 3 shows a modification of the screw shown in Fig. 2, and is a cross-sectional view of an embodiment in which the inside of the screw portion 10 is a hollow 12. The hollow inside 12 is provided not only to allow a guide for implanting the screw to pass through, but also to increase the surface area of the second material.

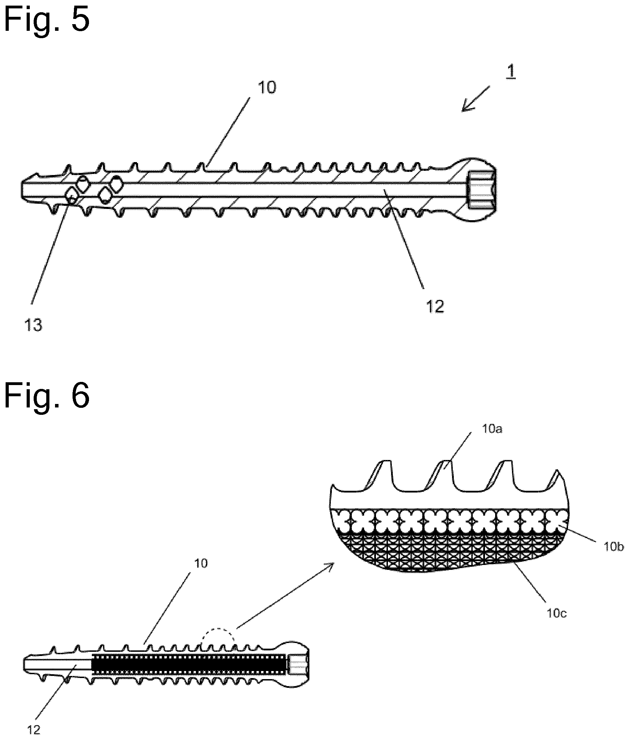

Figs. 4 and 5 show a modification of the screw shown in Fig. 2, and show an embodiment in which the screw portion 10 has a hollow 12 on the inside, and is provided with windows 13 that allow the hollow inside 12 of the screw to communicate with the outside. The windows 13 are provided to increase the surface area of the second material.

Fig. 6 shows an embodiment in which the second material forming the screw portion 10 is in the form of a different alloy powder. As shown in the partially enlarged cross-sectional view in the upper right portion of Fig. 6, the screw portion 10 is composed of a solid outer wall 10a formed by melting a plurality of powder particles made of the second material, and porous portions 10b and 10c formed of a plurality of powder particles made of the second material, wherein the solid outer wall 10a, and the porous portions 10b and 10c are laminated. The sintered portion 10c is a sintered portion of a side wall that is seen through the hollow inside of the screw portion 10. While the porous portions 10b and 10c are positioned under the solid outer wall 10a in the illustrated embodiment, an outer wall may be provided as a porous portion, and a solid portion may be provided under the outer wall. To laminate the porous portion and the solid portion, compression molding, sintering, diffusion bonding, additive manufacturing using an electron beam or laser, metal injection molding, spark plasma sintering, or a combination of these methods can be suitably used.

In this embodiment, the presence of sintered portions containing the second material as powder particles increases the surface area of the second material to increase the ratio of the surface area of the second material to the surface area of the first material. As shown in the results of the examples described below, the antimicrobial property improves as the ratio of the surface area of the second material increases.

Furthermore, while the screw portion 10 has the hollow 12 on the inside in the illustrated embodiment, it may also be solid inside. When the screw portion 10 is solid inside, the screw portion 10 contains a large amount of the laminated portion having the sintered portion 10b formed by sintering a plurality of powder particles made of the second material, and therefore, the surface area of the second material can be further increased to improve the antimicrobial property.

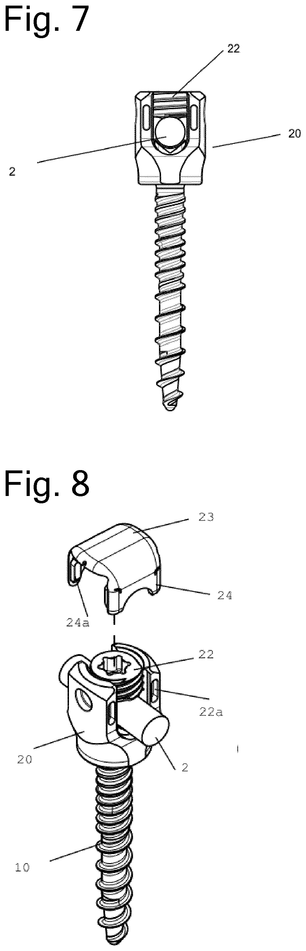

Fig. 7 shows an embodiment in which a set screw 22 is screwed on the head portion 20 to support the rod 2. The head portion 20 of the screw contains the second material, and the set screw 22 screwed on the head portion 20 contains the first material. The contact between the head portion 20 and the set screw 22 causes a short circuit between the alloys having a potential difference to form an electric circuit. In this embodiment, the screw portion 10 may be an integral type in which it is integrally formed with the head portion 20, using the same material, or a pivotally movable type in which it is formed independently of the head portion 20, using the same or a different material.

Fig. 8 shows an embodiment in which the set screw 22 for supporting the rod 2 is screwed on the head portion 20, and the head portion 20 is covered with a cap 23. The cap 23 is preferably made of a material different from that of the head portion 20. For example, when the head portion 20 is made of the first material, at least a surface of the cap 23 that contacts the head portion 20 is preferably made of the second material. In the illustrated embodiment, preferably, the cap 23 has a leg portion 24 having protrusions 24a that are fit into fitting holes 20a of the head portion 20, and the surface of the leg portion 24 is made of the second material.

EXAMPLES

The present invention will be hereinafter described in detail with examples; however, the invention is not limited to these examples.

[Example 1]

(Preparation of Samples)

A commercially available biocompatible titanium alloy (titanium content: 88 to 91 mass%) and a commercially available biocompatible cobalt-chromium alloy (cobalt content: 58 to 69 mass%, chromium content: 26 to 30 mass%) were processed into 3-mm-thick coin-shaped samples (only the titanium alloy of Sample No. 3 was processed into a flat plate-shaped sample) having the various areas shown in Table 1, and these samples were laminated in the various combinations shown in Table 1 and shorted to each other. As comparative samples, a 1-mm-thick flat plate-shaped polyethylene sample (control), a 3-mm-thick coin-shaped sample made of the cobalt-chromium alloy (Comparison 1), and a 3-mm-thick coin-shaped sample made of the titanium alloy (Comparison 2) were similarly prepared.

[Table 1]

Table 1 Sample Conditions

| Sample No. | Area Ratio* | Surface Area/mm$^2$ | |
| --- | --- | --- | --- |
| | | Titanium Alloy | Cobalt-Chromium Alloy |
| 1 | 1.0 | 518 | 518 |
| 2 | 3.3 | 1696 | 518 |
| 3 | 10.8 | 5600 | 518 |
| 4 | 0.2 | 103 | 518 |
| Comparison 1 | - | - | 518 |
| Comparison 2 | - | 518 | - |
| Control | - | Polyethylene | |
| * Area Ratio = Surface Area of Titanium Alloy/Surface Area of Cobalt-Chromium Alloy | | | |

(Antimicrobial Property Test)

An antimicrobial property was evaluated with reference to JIS Z 2801: Antibacterial products - Test for antibacterial activity and efficacy.

Each of the samples shown in Table 1 was placed in a petri dish, and 40 μl of *Staphylococcus aureus* (NBRC 12732) suspended in a nutrient broth (1/100) was added dropwise. A polyethylene film was placed over the test bacterial suspension added. The suspension was cultured at 35°C for 24 hours, and then cells were washed out from the sample. The resulting wash-out suspension was serially diluted in a 10-fold dilution series and cultured at 35°C for 24 hours using the agar plate culture method, and the number of viable cells was calculated from the number of colony forming units (CFUs) formed. The difference between the logarithmic value of the number of viable cells on the polyethylene sample as a control after 24-hour contact (Nc) and the logarithmic value of the number of viable cells on each sample (N) was determined as the antimicrobial activity value.

[Math. 1]

$$\text{Antimicrobial activity value} = \log(Nc/N)$$

Table 2 shows the number of viable cells and the antimicrobial activity value for each sample. A sample having an antimicrobial activity value of 2.0 or more was determined to have antimicrobial efficacy. The number of viable cells decreased (the antimicrobial activity value increased) as the area ratio (the surface area of the sample made of the titanium alloy/the surface area of the sample made of the cobalt-chromium alloy) increased.

[Table 2]

Table 2 Results of Antimicrobial Property Test

| Sample | Number of Viable Cells/CFU/Coupon | Antimicrobial Activity Value |
|---|---|---|
| Control: Polyethylene | $1.1 \times 10^6$ | - |
| 1 | $6.8 \times 10^3$ | 2.21 |
| 2 | $2.4 \times 10^3$ | 2.66 |
| 3 | $4.0 \times 10^2$ | 3.44 |
| Comparison 1 | $9.2 \times 10^3$ | 2.08 |

[Example 2]

(Preparation of Samples)

An implant device made of the titanium alloy and the cobalt-chromium alloy (the area ratio of the titanium alloy to the cobalt-chromium alloy was set to 0.89) was prepared (implant device 1). As a comparative example, an implant device made of only the titanium alloy having completely the same shape was similarly prepared (implant device 2).

[Table 3]

Table 3 Composition of Implant Device

| Implant Device No. | Area Ratio* | Combination of Materials | |
|---|---|---|---|
| | | Titanium Alloy | Cobalt-Chromium Alloy |
| 1 | 0.89 | ○ | ○ |
| 2 | --- | ○ | |
| * Area Ratio = Surface Area of Member Made of Titanium Alloy/Surface Area of Member Made of Cobalt-Chromium Alloy | | | |

**EP 3 424 449 A1**

(Antimicrobial Property Test)

Each of the implant devices was placed in a polypropylene tube, and immersed in 1 ml of *Staphylococcus aureus* (NBRC 12732) suspended in a nutrient broth (1/100). After 24 hours of culture at 35°C, the culture medium was collected. The collected culture medium was serially diluted in a 10-fold dilution series and cultured at 35°C for 24 hours using the agar plate culture method, and the number of viable cells was determined from the number of colony forming units (CFUs) formed.

Table 4 shows the number of viable cells and the antimicrobial activity value for each implant device. In the implant device 1 in which the area ratio of the titanium alloy to the cobalt-chromium alloy was appropriately set, the number of viable cells significantly decreased. In contrast, in the implant device 2 made of the titanium alloy only, a decrease in the number of viable cells was not observed.

[Table 4]

Table 4 Results of Antimicrobial Property Test for Implant Device

| Implant Device No. | Number of Viable Cells/CFU/Coupon | Antimicrobial Activity Value |
| --- | --- | --- |
| Polyethylene | $7.6 \times 10^7$ | - |
| 1 | $1.2 \times 10^5$ | 2.80 |
| 2 | $7.0 \times 10^7$ | 0.04 |

**Claims**

1. An antimicrobial implant device made of a first material and a second material, wherein
   at least a surface of the first material comprises an antimicrobial metal;
   at least a surface of the second material is nobler than the first material;
   an electric circuit is formed between the first material and the second material when the device is implanted in vivo for use; and
   ions of the antimicrobial metal are eluted from the first material under a presence of an electrolyte to impart an antimicrobial property to a surface and/or a surrounding area of the first material.

2. The antimicrobial implant device according to claim 1, wherein the first material comprises at least one antimicrobial metal selected from cobalt, silver, zinc, copper, tungsten, magnesium, nickel and phosphorus.

3. The antimicrobial implant device according to claim 1 or 2, wherein the first material is a cobalt-based alloy.

4. The antimicrobial implant device according to claim 3, wherein the cobalt-based alloy comprises cobalt in an amount of 29 to 69 mass%.

5. The antimicrobial implant device according to claim 3 or 4, wherein the cobalt-based alloy is a cobalt-chromium alloy.

6. The antimicrobial implant device according to claim 5, wherein the cobalt-chromium alloy comprises chromium in an amount of 18 to 30 mass%.

7. The antimicrobial implant device according to any one of claims 1 to 6, wherein the second material is titanium or a titanium-based alloy.

8. The antimicrobial implant device according to claim 7, wherein the titanium or the titanium-based alloy comprises titanium in an amount of 68 to 100 mass%.

9. The antimicrobial implant device according to any one of claims 1 to 8, wherein a ratio of a surface area of the second material to a surface area of the first material (second material/first material) is in a range of 0.2 to 10.8.

10. The antimicrobial implant device according to any one of claims 2 to 9, wherein cobalt ions are eluted from the first material in an amount of 7 nmol/l to 81 μmol/l.

11. The antimicrobial implant device according to any one of claims 1 to 10, wherein
the antimicrobial implant device is a screw comprising a screw portion and a head portion;
the head portion of the screw comprises the first material; and
the screw portion of the screw comprises the second material.

12. The antimicrobial implant device according to claim 11, wherein the screw is a hollow screw having a hollow on the inside of the screw portion.

13. The antimicrobial implant device according to claim 11 or 12, wherein the screw is a hollow screw having a hollow on the inside of the screw portion and a window that allows the hollow inside to communicate with the outside of the screw.

14. The antimicrobial implant device according to any one of claims 11 to 13, wherein the screw portion comprises
a porous section comprising the second material; and
a solid section comprising the second material.

15. The antimicrobial implant device according to any one of claims 1 to 10, wherein
the antimicrobial implant device is a screw comprising a screw portion and a head portion; and
the head portion of the screw comprises the second material; and
the antimicrobial implant device further has a set screw comprising the first material that is screwed on the head portion and/or a cap comprising the first material that is fit on the head portion;
wherein, optionally, the head portion comprises
a porous section comprising the second material; and
a solid section comprising the second material.

# Fig. 1

# Fig. 2

# Fig. 3

# Fig. 4

Fig. 5

Fig. 6

# Fig. 7

22

2

20

# Fig. 8

23

24

24a

22

22a

20

2

10

## INTERNATIONAL SEARCH REPORT

| International application No. |
|---|
| PCT/JP2017/007787 |

A. CLASSIFICATION OF SUBJECT MATTER
*A61B17/58*(2006.01)i, *A61L27/04*(2006.01)i, *A61L27/40*(2006.01)i

According to International Patent Classification (IPC) or to both national classification and IPC

B. FIELDS SEARCHED

Minimum documentation searched (classification system followed by classification symbols)
A61B17/58, A61L27/04, A61L27/40

Documentation searched other than minimum documentation to the extent that such documents are included in the fields searched
Jitsuyo Shinan Koho          1922-1996   Jitsuyo Shinan Toroku Koho   1996-2017
Kokai Jitsuyo Shinan Koho    1971-2017   Toroku Jitsuyo Shinan Koho   1994-2017

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X | US 2012/0123485 A1 (DEHNAD, Houdin), | 1-2,9 |
| Y | 17 May 2012 (17.05.2012), | 3-8,10 |
| A | paragraph [0102]; fig. 7A to 7C | 11-16 |
| | & US 2013/0144204 A1     & US 2014/0288607 A1 | |
| | & US 2015/0320989 A1     & WO 2012/064402 A1 | |
| | & EP 3037066 A1 | |
| | | |
| Y | JP 5175185 B2 (ArgentumCidalElectrics, Inc.), | 3-6,10 |
| | 03 April 2013 (03.04.2013), | |
| | paragraph [0044] | |
| | & WO 2007/005842 A2 | |
| | page 11, lines 8 to 15 | |
| | & JP 2009-500084 A        & US 2006/0004431 A1 | |
| | & US 2010/0100188 A1      & US 2010/0198357 A1 | |
| | & US 2012/0316655 A1      & US 2014/0094750 A1 | |
| | & US 2015/0182645 A1      & US 2016/0325094 A1 | |
| | & CA 2613121 A1 | |

[X] Further documents are listed in the continuation of Box C.          [ ] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 19 May 2017 (19.05.17) | 30 May 2017 (30.05.17) |

| Name and mailing address of the ISA/ | Authorized officer |
|---|---|
| Japan Patent Office | |
| 3-4-3,Kasumigaseki,Chiyoda-ku, | |
| Tokyo 100-8915,Japan | Telephone No. |

Form PCT/ISA/210 (second sheet) (January 2015)

**INTERNATIONAL SEARCH REPORT**

International application No.

PCT/JP2017/007787

C (Continuation).   DOCUMENTS CONSIDERED TO BE RELEVANT

| Category* | Citation of document, with indication, where appropriate, of the relevant passages | Relevant to claim No. |
|---|---|---|
| Y | JP 2002-536048 A  (Synthes AG. Chur), 29 October 2002 (29.10.2002), paragraphs [0008] to [0019]; fig. 1 to 4 & US 2002/0029043 A1 paragraphs [0019] to [0025]; fig. 1 to 3 & WO 2000/045724 A1 | 7-8 |
| A | JP 08-506027 A  (Implemed, Inc.), 02 July 1996 (02.07.1996), claims; fig. 5 to 11 & WO 1994/011058 A1 claims; fig. 5 to 11 & US 6287484 B1          & US 6060000 A & US 5725817 A          & US 5498248 A & US 5322520 A          & WO 1999/046780 A1 | 1-16 |
| A | JP 2001-258894 A  (Chun-Tek Lee), 25 September 2001 (25.09.2001), fig. 5 to 7 & US 2001/0023350 A1 fig. 5 to 7 & WO 2001/067998 A1     & EP 1133951 A2 & CN 1313068 A | 15-16 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 5175185 B